# EUROPEAN PATENT APPLICATION

(11) **EP 4 807 003 A1**
(43) Date of publication of application: **16.09.2026**
(21) Application number: 25162697.4
(22) Date of filing: 10.03.2025
(51) Int. Cl.: C12N 15/113

(54) **METHOD AND COMPOUNDS TO ADDRESS AGEING**

(71) Applicant: Resalis Therapeutics S.r.l., 10121 Torino (IT)
(72) Inventor: Kauppinen, Sakari, 10121 Torino (IT); Tomasini, Simone, 10121 Torino (IT); Panella, Riccardo, 10121 Torino (IT)
(74) Representative: Rigamonti, Dorotea

(57) **Abstract**

It forms an object of the present invention a method for addressing senescence and aging, comprising administering an effective amount of an inhibitor of miR-22 to a subject in need thereof.

## Description

### FIELD OF THE INVENTION

The presence invention is focused on an approach targeting a non-coding RNA to address ageing, wherein microRNA-22 is targeted using a modified antisense oligonucleotide compound.

### BACKGROUND

MicroRNAs (miRNA or miR) are nucleic acid molecules that regulate the expression of target genes. MiRNAs are typically short (typically 18-24 nucleotides) and act as repressors of target mRNAs by promoting their degradation, when their sequences are perfectly complementary, and/or by inhibiting translation, when their sequences contain mismatches. Functional analyses of miRNAs have revealed that these small, non-coding RNAs contribute to different physiological and metabolic processes.

WO2006137941 describes a method and a composition for reducing or inhibiting the cell growth, a method for inducing or increasing the cell growth, a method for decreasing the cell survival rate, a method for increasing the cell survival rate, a method for increasing apoptosis of cells, and a method for inhibiting apoptosis of cells, which methods comprise the step of introducing an effective amount of a specific synthetic miRNA molecule to cells. More particularly, it is described that (hsa-)miR-22 is a miRNA which significantly decreases the number of livings A549 cells (human lung cancer cells) and significantly increases the percentage of apoptotic cells. Further, a method for treating cancer in a subject, which method uses such a function and comprises the step of administration of an effective amount of a synthetic miRNA molecule corresponding to (hsa-)miR-22 to the subject, is described.

The mechanism and meaning of the induction of cellular senescence due to replication, including the relationship with aging of individuals, have been controversial for a long time. This is because cellular senescence is considered to be an important biological defense mechanism along with apoptosis, and a new application of cellular senescence to cancer therapy is therefore expected. That is, this is because, similarly to apoptosis, cellular senescence is involved in the cell growth and a protection mechanism against abnormal cells. Thus, elucidation of genes involved in cellular senescence has been demanded.

EP2518144 describes that the gene transcript of (hsa-)miR-22 is highly expressed in senescent human fibroblasts.

WO2019178411 and WO2019178410 describe compositions comprising miR-22 inhibitors, among these LNA10 (SEQ ID NO: 8), and their use in treating metabolic disorders.

There are currently no approved or effective therapeutic approaches for treatment of senescence. Ageing is considered irreversible and the only few options available to slow down or revert this process are based on dietary intervention or pharmacological targeting of specific proteins involved in the mTOR molecular pathway.

### DESCRIPTION

It forms an object of the present invention a method to address senescence and ageing by targeting miR-22.

### DESCRIPTION OF THE DRAWINGS

**Figure 1****:** inhibition of miR-22 in BJ fibroblasts while reaching replicative senescence; (VHL= control; LNA10 = 0,5 µM LNA10).
**Figure 2****:** LNA10 treatment effect of replicative senescence on middle (A) and late (B) passage BJ fibroblasts.
**Figure 3****:** miR-22 and miR-22 direct targets expression fold change over time.
**Figure 4****:** (A, B, C) senescence marker expression.
**Figure 5****:** (A, B, C, D) inflammatory chemokines and cytokines expression.
**Figure 6****:** (A, B, C, D) inflammatory chemokines and cytokines expression.
**Figure 7****:** (A, B) chromatin modulators expression.

### DETAILED DESCRIPTION

The here described approach targets miR-22 to treat senescence and ageing. Surprisingly, this approach revealed effective in modulating several genes implicated in molecular pathways that are associated with senescence. The experiments performed to evaluate tolerability and safety in rodents and non-human primates demonstrate this therapy is safe and well tolerated.

Without being bound by theory, mature miRNAs are believed to be generated by pol II or pol III and arise from initial transcripts termed pri-miRNAs. These pri-miRNAs are frequently several thousand bases long and are therefore processed to make much shorter mature miRNAs. These pri-miRNAs may be multicistronic and result from the transcription of several clustered sequences that organize what may develop into many miRNAs. The processing to yield miRNAs may be two-steps. First, pri-miRNAs may be processed in the nucleus by the RNase Drosha into about 70- to about 100-nucleotide hairpin-shaped precursors (pre-miRNAs). Second, after transposition to the cytoplasm, the hairpin pre-miRNAs may be further processed by the RNase Dicer to produce a double-stranded miRNA. The mature miRNA strand may then be incorporated into the RNA-induced silencing complex (RISC), where it may associate with its target mRNAs by base-pair complementarity and lead to suppression of protein expression. The other strand of the miRNA duplex that is not preferentially selected for entry into a RISC silencing complex is known as the passenger strand or minor miRNA or star (*) strand. This strand may be degraded. It is understood that, unless specified, as used herein a miRNA may refer to pri- and/or pre- and/or mature and/or minor (star) strand and/or duplex version of miRNA.

In some embodiments, miRNA genes may be located within introns of protein-coding genes or within introns or exons of noncoding transcriptional units. The expression of intronic miRNAs may coincide with that of the hosting transcriptional units because they are typically oriented in the same direction and are co-ordinately expressed with the pre-mRNAs in which they reside.

In some embodiments, miRNAs may bind to sequences within the 3' untranslated region (3'UTR) of target gene transcripts. In some embodiments, miRNAs may bind to sequences outside of the 3'UTR of target gene transcripts. In some embodiments, miRNAs may bind to both within and outside the 3'UTR of target gene transcripts.

In some embodiments, nucleotide pairing between the second and seventh nucleotides of the miRNA (the miRNA seed sequence) and the corresponding sequence along the target 3'UTR (seed match) may occur for target recognition. Accordingly, the binding between miRNA and target may comprise about a 5-nucleotide base pairing. Additionally, the binding between miRNA and target may comprise more than a 5-nucleotide base pairing. In some embodiments, the binding between a miRNA and the gene that it regulates may be mediated by the miRNA binding up to 2, up to 4, up to 6, up to 8, or up to 10 sites of the target nucleic acid.

MiR-22 is highly conserved across many vertebrate species, including chimp, mouse, rat, dog and horse. This level of conservation suggests functional importance. MiR-22 was previously identified as having a role in erythrocyte maturation and later as having a role in oncogenesis. MiR-22 directly targets phosphatase and tensin homolog (PTEN) and tet methyl cytosine dioxygenase (TET) to promote tumorigenesis, metastasis, and metabolic disorders. In some embodiments, the nucleic acids of the present invention increase the activity and/or expression of PTEN and/or TET2.

The predicted miR-22 hairpin precursor is contained entirely within exon 2 of a noncoding transcript, CI7orf91, and the splicing pattern is generally conserved in human and mouse, despite the lack of protein-coding potential. See Rodriguez et al., Identification of mammalian microRNA host genes and transcription units. Genome Res. 2004 Oct; 14(10A): 1902-10. Deletion of exon 2 of C17orf91 encompassing mir-22 in mouse models has revealed that miR-22 may play a role in cardiac hypertrophy and remodelling by targeting SIRT1 (NAD-dependent deacetylase sirtuin-1), HDAC4 (histone deacetylase 4), PURB (purine-rich element binding protein B) and PTEN. See Gurha et al., Targeted deletion of microRNA-22 promotes stress-induced cardiac dilation and contractile dysfunction. Circulation. 2012 Jun 5;125(22):2751-61 ; Huang et al., MicroRNA-22 regulates cardiac hypertrophy and remodelling in response to stress. Circ Res. 2013 Apr 26;112(9):1234-43 .

In embodiments, an inhibitor of miRNA is a nucleic acid that acts as an antisense oligonucleotide. Nucleic acids of the present invention can include ribonucleotides or deoxyribonucleotides or a combination thereof. Nucleic acids of the present invention may have at least one chemical modification (non-limiting examples are sugar or backbone modifications, e.g., a Locked Nucleic Acid (LNA)).

In embodiments, the sequence of a nucleic acid that inhibits miR-22 is conserved across species. In embodiments, the sequence of the nucleic acid is complementary, in part, to the sequence of human miR-22. In embodiments, the inhibitor is selected to reduce the expression and/or activity of the target miR-22 in a cell or a subject.

In embodiments, nucleic acids of the present invention inhibit human miR-22. In embodiments, human miR-22 comprises or consists of AAGCUGCCAGUUGAAGAACUGU (SEQ ID NO: 1).

In embodiments, the nucleic acids of the present invention are about 8 to about 20 residues in length (e.g., about 8-18, or about 8-16, or about 8-14, or about 8-12, or about 8-10, or about 10-18, or about 10-16, or about 10-14, or about 10-12 residues in length). In embodiments, the nucleic acids are about 8, or about 9, or about 10, or about 11, or about 12 residues in length.

In embodiments, nucleic acids of the present invention bind with complete homology to a portion of miR-22. For example, a nucleic acid of the present invention may be 18 residues in length and each of the 18 residues is complementary to a nucleotide of miR-22. Alternately, the nucleic acids of the present invention bind with complete homology to a portion of miR-22. For example, for a nucleic acid of the present invention may be 16 residues in length and only 15 or fewer of the residues are complementary to a nucleotide of miR-22.

In embodiments, a nucleic acid of the present invention differs from a portion of miR-22 at one position, at two positions, at three positions, at four positions, at five positions, or at more than five positions.

In embodiments, a nucleic acid of the present invention binds to miR-22 with sufficient affinity to inhibit it. In embodiments, the nucleic acids bind to miR-22 with a high affinity (e.g. nM affinity). Thus, a nucleic acid of the present invention binds to miR-22 with a high affinity even if it differs from a portion of miR-22 at one or more positions.

A nucleic acid of the present invention may have a sequence comprising tggcagct (SEQ ID NO: 2) and comprising at least one locked nucleic acid (LNA) modification.

In a locked nucleic acid (LNA) the nucleic acid's ribose moiety is modified with an extra bridge connecting the 2' oxygen and 4' carbon, which locks the ribose in the 3'-endo conformation.

Nucleic acids of the present invention, and comprising LNAs, provide for cost-effective agents that can be delivered efficiently and possess sufficient bioavailability for the treatment and prevention of various disorders.

In embodiments, a nucleic acid of the present invention includes at least 2 LNA modifications towards its 3' end (e.g., about 2, or about 3, or about 4, or about 5 modifications towards its 3' end). In embodiments, the nucleic acid comprises tggcagct (SEQ ID NO: 2), and at least 6 LNA modifications or at least 8 LNA modifications.

For example, the nucleic acid comprises LNA modifications at positions 7 and 8 of tggcagct (SEQ ID NO: 2). In embodiments, a nucleic acid of the present invention includes no more than 4 sequential LNA modifications (e.g., only 2, or 3, or 4 sequential LNA modifications). In embodiments, a nucleic acid of the present invention includes no more than 3 sequential unmodified residues (e.g., only 3, or 2, or 1 unmodified residues). In embodiments, a nucleic acid of the present invention includes no less than 4 LNA modifications. In embodiments, the nucleic acid comprises about 8 to about 12 LNA modifications, e.g., about 8, or about 9, or about 10, or about 11, or about 12 LNA modifications.

In embodiments, the nucleic acid comprises or consists of a nucleic acid having a sequence selected from: tggcagct (SEQ ID NO: 2), cttcaactggcagct (SEQ ID NO: 3), tcttcaactggcagct (SEQ ID NO: 4), ttcttcaactggcagct (SEQ ID NO: 5), and gttcttcaactggcagct (SEQ ID NO: 6). In embodiments, the nucleic acid comprises a nucleic acid having a sequence selected from: tggcagct (SEQ ID NO: 2), cttcaactggcagct (SEQ ID NO: 3), tcttcaactggcagct (SEQ ID NO: 4), ttcttcaactggcagct (SEQ ID NO: 5), and gttcttcaactggcagct (SEQ ID NO: 6) and comprising about 8 to about 12 LNA modifications, e.g., about 8, or about 9, or about 10, or about 11, or about 12 LNA modifications.

In embodiments, the nucleic acid comprises or consists of cttcaactggcagct (SEQ ID NO: 3), and at least 6 LNA modifications, at least 8 LNA modifications, or at least 10 LNA modifications. In embodiments, the nucleic acid comprises or consists of cttcaactggcagct (SEQ ID NO: 3), and 8 LNA modifications, said modifications being at positions 1, 3, 6, 7, 10, 12, 14, and 15; for example, the nucleic acid comprises or consists of the sequence CtTcaACtgGcAgCT (SEQ ID NO: 7), where capital letters are LNA modified and lower case are unmodified. In embodiments, the nucleic acid comprises or consists cttcaactggcagct (SEQ ID NO: 3), and 10 LNA modifications, said modifications being at positions 1, 2, 3, 6, 7, 10, 11, 12, 14, and 15; for example, the nucleic acid comprises or consists of the sequence CTTcaACtgGCAgCT (SEQ ID NO: 8), where capital letters are LNA modified and lower case are unmodified.

In embodiments, the nucleic acid comprises or consists of tcttcaactggcagct (SEQ ID NO: 4), and at least 8 LNA modifications, at least 10 LNA modifications, or at least 11 LNA modifications. In embodiments, the nucleic acid comprises or consists of tcttcaactggcagct (SEQ ID NO: 4), and 10 LNA modifications, said modifications being at positions 1, 2, 4, 8, 10, 11, 12, 13, 15, and 16; for example, the nucleic acid comprises or consists of the sequence TCtTcaaCtGGCAgCT (SEQ ID NO: 10), where capital letters are LNA modified and lower case are unmodified. In embodiments, the nucleic acid comprises or consists of tcttcaactggcagct (SEQ ID NO: 4), and 11 LNA modifications, said modifications being at positions 1, 2, 4, 5, 6, 8, 11, 12, 13, 15, and 16; for example, the nucleic acid comprises or consists of the sequence TCtTCAaCtgGCAgCT (SEQ ID NO: 9), where capital letters are LNA modified and lower case are unmodified.

In embodiments, the nucleic acid comprises or consists of tcttcaactggcagct (SEQ ID NO: 4), and 11 LNA modifications, said modifications being at positions 1, 2, 4, 5, 6, 9, 11, 12, 13, 15, and 16; for example, the nucleic acid comprises or consists of the sequence TCtTCAacTgGCAgCT (SEQ ID NO: 11), where capital letters are LNA modified and lower case are unmodified.

In embodiments, the nucleic acid comprises or consists of ttcttcaactggcagct (SEQ ID NO: 5), and at least 10 LNA modifications or at least 11 LNA modifications. In embodiments, the nucleic acid comprises or consists of ttcttcaactggcagct (SEQ ID NO: 5), and 11 LNA modifications, said modifications being at positions 1, 2, 5, 6, 7, 10, 12, 13, 14, 16, and 17; for example, the nucleic acid comprises or consists of the sequence TTctTCAacTgGCAgCT (SEQ ID NO: 12), where capital letters are LNA modified and lower case are unmodified.

In embodiments, the nucleic acid comprises or consists of gttcttcaactggcagct (SEQ ID NO: 6), and at least 9 LNA modifications or at least 10 LNA modifications. In embodiments, the nucleic acid comprises or consists of gttcttcaactggcagct (SEQ ID NO: 6), and 9 LNA modifications, said modifications being at positions 1, 2, 6, 10, 13, 14, 16, 17, and 18; for example, the nucleic acid comprises or consists of the sequence GTtctTcaaCtgGCaGCT (SEQ ID NO: 13), where capital letters are LNA modified and lower case are unmodified.

In embodiments, the nucleic acid comprises or consists of a nucleic acid having a sequence selected from: tggcagct (SEQ ID NO: 2), gttcttcaactggcagct (SEQ ID NO: 6), and comprising about 8 to about 12 LNA modifications, e.g., about 8, or about 9, or about 10, or about 11, or about 12 LNA modifications.

In embodiments, the nucleic acid comprises or consists of gttcttcaactggcagct (SEQ ID NO: 6), and at least 9 LNA modifications, or at least 10 LNA modifications, or at least 11 LNA modifications, or at least 12 LNA modifications.

In an embodiment, the nucleic acid comprises at least 10, or at least 11, or at least 12 locked nucleic acid (LNA) modifications.

In an embodiment, the nucleic acid comprises 10 or 11 locked nucleic acid (LNA) modifications.

In an embodiment, the nucleic acid consists of gttcttcaactggcagct (SEQ ID NO: 6), and comprises 10 or 11 modifications, said modifications being at least at positions 1, 2, 6, 11, 17, 18.

In an embodiment, the nucleic acid consists of gttcttcaactggcagct (SEQ ID NO: 6), and comprises 11 modifications, said modifications being at least at positions 1, 2, 5, 6, 11, 14, 17, 18.

In an embodiment, the nucleic acid consists of gttcttcaactggcagct (SEQ ID NO: 6), and at least one PS (Phosphorothioate) linkage has been substituted with one PO (Phosphine Oxide) linkage.

In an embodiment, the nucleic acid consists of gttcttcaactggcagct (SEQ ID NO: 6) and one PS linkage has been substituted with one PO linkage.

In an embodiment said PO linkage is at least at positions 6, 8, 10, or 14.

In an embodiment, said PO linkage is only one and it is at position 6, 10, or 14.

For example, the nucleic acid comprises or consists of the sequence GTTctTcAAcTGgCAgCT (SEQ ID NO: 16), where capital letters are LNA modified and lower case are unmodified.

For example, the nucleic acid comprises or consists of the sequence GTtcTTCaAcTGgCagCT (SEQ ID NO: 17), where capital letters are LNA modified and lower case are unmodified.

For example, the nucleic acid comprises or consists of the sequence GTtcTTcAaCTGgCAgCT (SEQ ID NO: 18), where capital letters are LNA modified and lower case are unmodified.

For example, the nucleic acid comprises or consists of the sequence GTtCtTcAaCTggcAgCT (SEQ ID NO: 19), where capital letters are LNA modified and lower case are unmodified.

For example, the nucleic acid comprises or consists of the sequence GTtcTTcAaCtgGCAgCT (SEQ ID NO: 20), where capital letters are LNA modified and lower case are unmodified.

In one embodiment, an expression vector for expressing a nucleic acid of the present invention comprises a promoter operably linked to a polynucleotide encoding the nucleic acid of the present invention. The phrase operably linked or under transcriptional control as used herein means that the promoter is in the correct location and orientation in relation to a polynucleotide to control the initiation of transcription by RNA polymerase and expression of the polynucleotide.

As used herein, a promoter refers to a DNA sequence recognized by the synthetic machinery of the cell, or introduced synthetic machinery, required to initiate the specific transcription of a gene. Suitable promoters include, but are not limited to, RNA pol I, pol II, pol III, and viral promoters (e.g., human cytomegalovirus (CMV) immediate early gene promoter, the SV40 early promoter, and the Rous sarcoma virus long terminal repeat).

In certain embodiments, the promoter operably linked to a polynucleotide encoding a nucleic acid of the present invention can be an inducible promoter. Inducible promoters are known in the art and include, but are not limited to, the tetracycline promoter, the metallothionein IIA promoter, the heat shock promoter, the steroid/thyroid hormone/retinoic acid response elements, the adenovirus late promoter, and the inducible mouse mammary tumor virus LTR.

Methods of delivering expression constructs and nucleic acids to cells are known in the art and can include, by way of non-limiting example, calcium phosphate co-precipitation, electroporation, microinjection, DEAE-dextran, lipofection, transfection employing polyamine transfection reagents, cell sonication, gene bombardment using high velocity microprojectiles, and receptor-mediated transfection.

An aspect of the present invention provides a host cell comprising any herein-described nucleic acid of the present invention.

In another aspect, the present invention provides a pharmaceutical composition comprising any herein-described nucleic acid of the present invention and a pharmaceutically acceptable excipient or carrier.

Where clinical applications are contemplated, pharmaceutical compositions may be prepared in a form appropriate for the intended application. Generally, this will entail preparing compositions that are essentially free of pyrogens, as well as other impurities that could be harmful to humans or animals.

In one embodiment, a pharmaceutical composition comprises an effective dose of any herein-described nucleic acid of the present invention. An effective dose is an amount sufficient to affect a beneficial or desired clinical result. An effective dose may be from about 1 mg/kg to about 100 mg/kg, about 2.5 mg/kg to about 50 mg/kg, or about 5 mg/kg to about 25 mg/kg. The precise determination of what would be considered an effective dose may be based on factors individual to each patient, including their size, age, type of metabolic disorder, and nature of inhibitor or agonist (non-limiting examples include antagomir, expression construct, antisense oligonucleotide, polynucleotide duplex, etc.). Therefore, dosages can be readily ascertained by those of ordinary skill in the art from this disclosure and the knowledge in the art. For example, doses may be determined with reference Physicians' Desk Reference, 66th Edition, PDR Network; 2012 Edition (December 27, 2011), the contents of which are incorporated by reference in its entirety.

The here described approach has the advantage of providing a completely new strategy to address senescence and ageing. The here proposed approach is effective in modulating several genes implicated in molecular pathways that are associated with senescence.

### EXAMPLES

In order that the invention disclosed herein may be more efficiently understood, examples are provided below. These examples are for illustrative purposes only and are not to be construed as limiting the invention in any manner.

### Example 1: Design of LNA-modified antimiR-22 oligonucleotides

The oligonucleotides were designed to cover the seed sequence, contain between 8 nt and 20 nt in length, have a length-specific fraction of LNAs allowed and as high a binding affinity to miR-22 as possible.

Design elements included at least 8 LNA modifications in the construct. Further design elements included no more than 4 LNA modifications in a row. Further still design elements included no more than 3 unmodified residues in a row.

The oligonucleotides were designed to bind to miR-22 with sufficient affinity to inhibit it. Also, the oligonucleotides were designed to have limited or no self-binding affinity (e.g. no or limited duplex or fold structures).

In the below sequences (comparative), capital letters are LNA-modified and lower-case letters are unmodified; the orientations for the miR-22 (SEQ ID NO: 1) and for the anti-miR-22 oligonucleotides (SEQ ID NO: 2 and SEQ ID NO: 7 to SEQ ID NO: 13) are orientated 5' to 3'. The oligonucleotides of SEQ ID NO: 14 and SEQ ID NO: 15, orientated 5' to 3', are scrambled sequences and do not hybridize to the miR-22 (SEQ ID NO: 1).

**Table 1**

| | | |
|---|---|---|
| hsa-miR-22 | AAGCUGCCAGUUGAAGAACUGU | (SEQ ID NO: 1) |
| CRM0008 | TGGCAGCT | (SEQ ID NO: 2) |
| CRM0009 | CtTcaACtgGcAgCT | (SEQ ID NO: 7) |
| CRM0010 | CTTcaACtgGCAgCT | (SEQ ID NO: 8) |
| CRM0011 | TCtTCAaCtgGCAgCT | (SEQ ID NO: 9) |
| CRM0012 | TCtTcaaCtGGCAgCT | (SEQ ID NO: 10) |
| CRM0013 | TCtTCAacTgGCAgCT | (SEQ ID NO: 11) |
| CRM0014 | TTctTCAacTgGCAgCT | (SEQ ID NO: 12) |
| CRM0015 | GTtctTcaaCtgGCaGCT | (SEQ ID NO: 13) |
| CRM0016 | CGaATAgTtaGTAgCG | (SEQ ID NO: 14) |
| CRM0017 | FAM labelled-CGaATAgTtaGTAgCG | (SEQ ID NO: 15) |

In Table 1, capital letters are LNA-modified and lower-case letters are unmodified.

### Example 2: Assessment of in-vitro effect of antimiR-22 oligonucleotide compounds

A human fibroblasts cell lines, BJ, has been used for the experiments. Cells were plated at a density of 17.800 cell/cm². Cells were treated as follows:
1) Vehicle only;
2) 0,5 µM scramble LNA;
3) 0,5 µM LNA10

After 96h in colture, a β-gal assay was performed to assess cell viability.

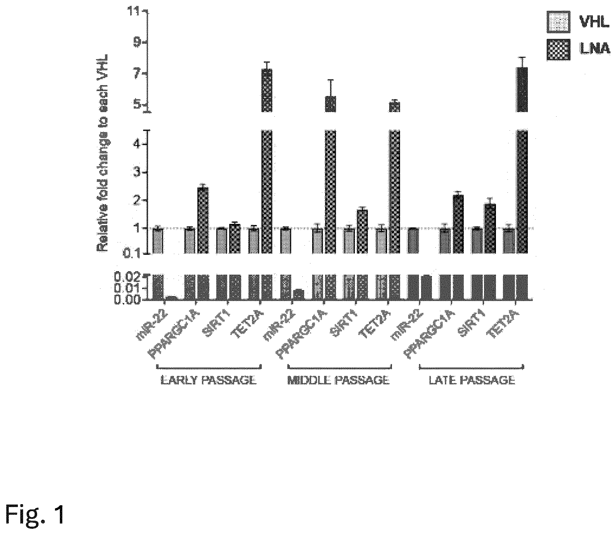

The expression of miR-22 and of miR-22 targets PPARGC1A, SIRT1, TET2A has been evaluated at early passage (P10), middle passage (P34) and late passage (P50). Results are reported in graph of Figure 1 and show that LNA10 almost completely inhibit miR-22. Moreover, expression of direct targets of miR-22 remains upregulated in fibroblasts from early to late passages.

BJ fibroblasts were then cultured in 10 cm dish, splitted and 10⁶ cells/ dish seeded every 4 days. Cells were cultured in complete growth media from P2 to P20. From P21 (62 days), cells were split in three culture condition:
1) Vehicle only (VHL);
2) 0,5 µM scramble LNA (SCR);
3) 0,5 µM LNA10 (LNA10).

β-gal staining of LNA treated and non-treated BJ cells with a fluorescent substrate for the enzyme at different cell passages was performed, demonstrating a progressive increase in the number of senescent cells that correlates with a flattening of the growth curve. LNA10 treatment is more efficacious in slowing aging at intermediate cell passages.

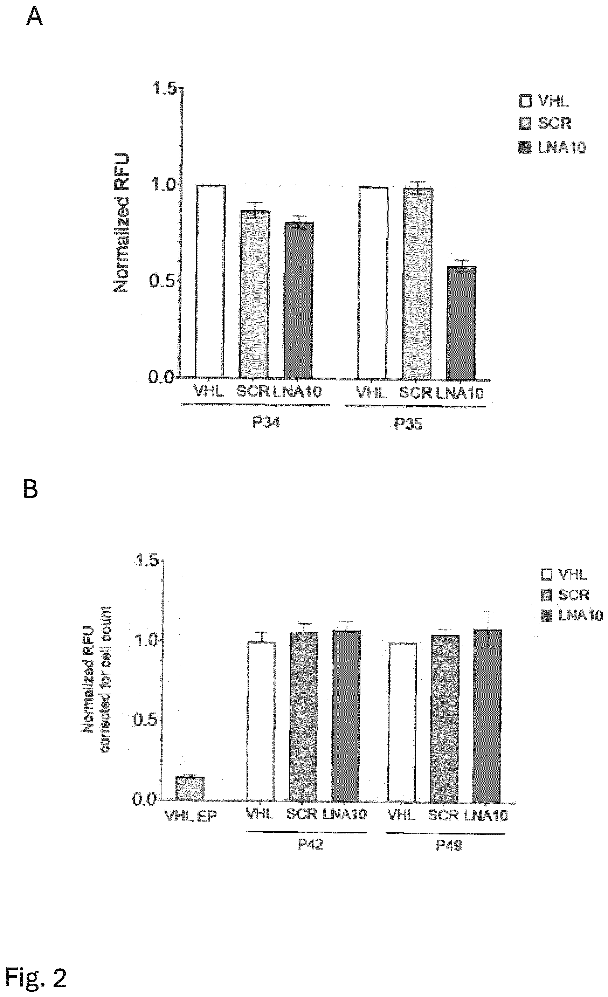

The graph in Figure 2A shows that on middle passage cells fluorescence is reduced for cells treated with LNA10 that are entering senescence compared to the non-treated controls. This is indicative of the fact that LNA10 may slow aging in skin fibroblasts.

The same experiment, performed on late passage cells, demonstrated that LNA10 does not revert senescence in fibroblasts, wherein senescent BJ fibroblasts do not show difference in fluorescence between treatment and controls, see Figure 2B.

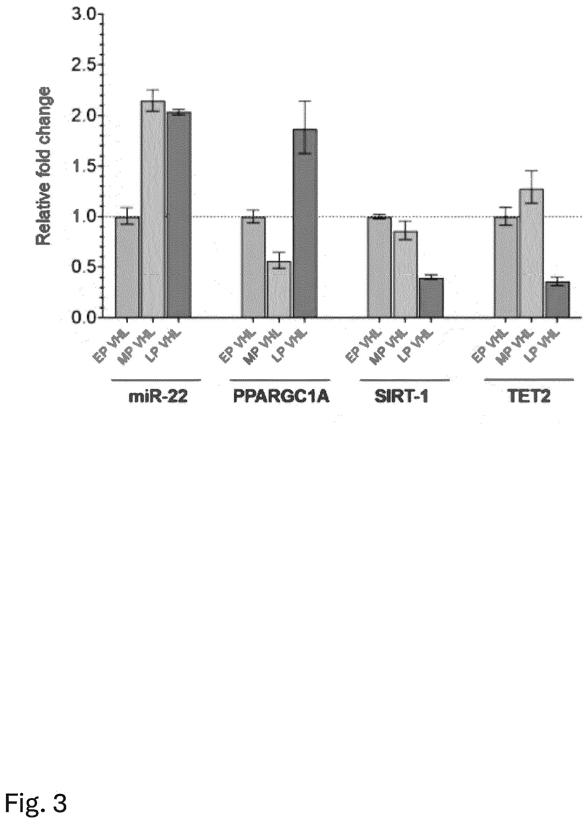

The levels of miR-22 and its direct targets were evaluated over time, at P10, P34 and P50. miR-22 levels double in cells approaching the senescence plateau (Figure 3). Some of miR-22 direct targets, SIRT-1 and TET2, are downregulated in senescent cells compared to early passages.

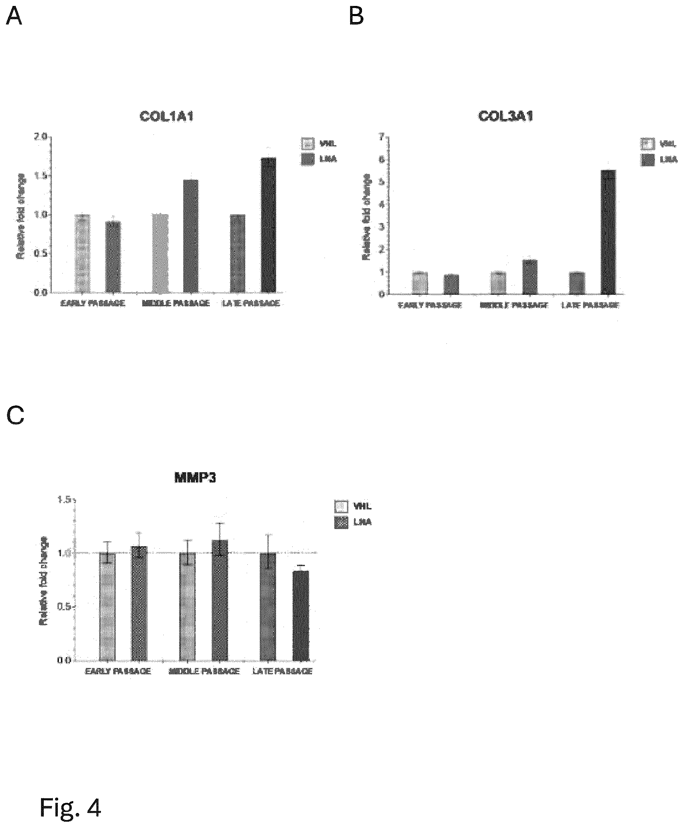

The expression levels of known senescence markers of the extracellular matrix were evaluated. Data, reported in Figure 4, shows that miR-22 inhibition mitigates and reverts the senescence-induced downregulation of collagen genes COL1A1 (Figure 4A), COL3A1 (Figure 4B), while miR-22 inhibition negatively modulates metalloproteases to slow the establishment of a senescent phenotype (Figure 4C).

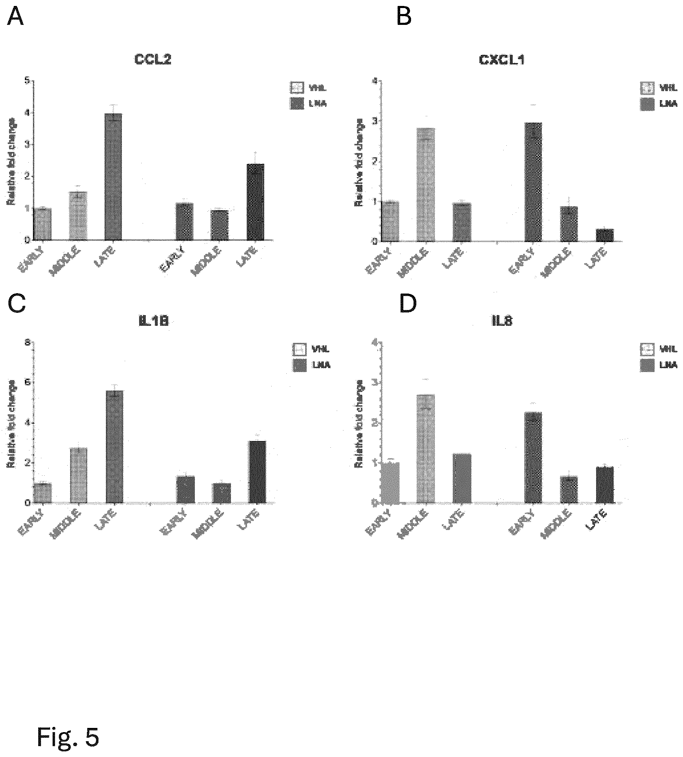

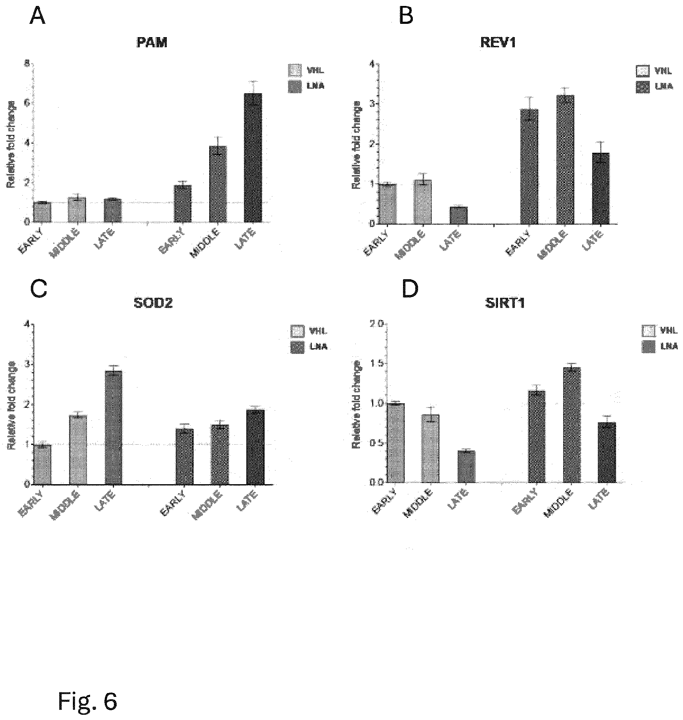

Senescence associated secretory phenotype (SASP) comprises a large group of inflammatory chemokines and cytokines, among them CCL2, CXCL1, IL-1B, IL-8. The levels of them were evaluated on BJ fibroblasts, treated or untreated with LNA10. Data are shown in Figure 5A (CCL2), B (CXCL1), C (IL-1B), D (IL-8). This panel shows that all of them are negatively modulated by LNA10, especially in middle passage cells. The establishment of the senescence phenotype is delayed in treated cells compared to control. Other senescence markers, namely PAM, REV1, SOD2 and SIRT1, have been evaluated following the same protocol. The results are in Figure 6A-D.

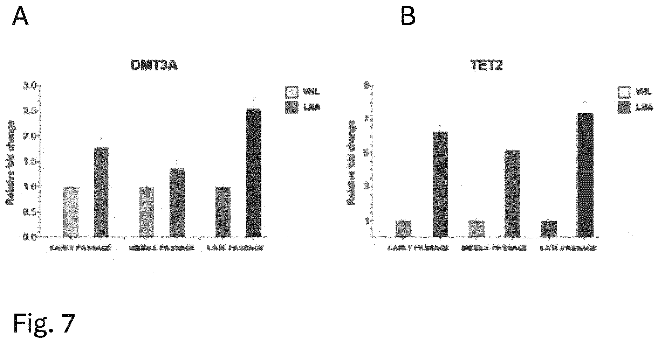

The impact on chromatin modulators DMT3A and TET2 has then been evaluated. Data, reported in Figure 7A (DMT3A) and B (TET2) shows an increase in their expression when BJ fibroblasts are treated with LNA10.

## Claims

1. A method for addressing senescence and aging, comprising administering an effective amount of an inhibitor of miR-22 to a subject in need thereof.

2. The method of claim 1, wherein said subject is a non-geriatric subject.

3. The method of claim 1, wherein the senescence-induced downregulation of expression of collagen genes COL1A1 and COL3A1 is reverted following administration of said inhibitor.

4. The method of claim 1, wherein the expression and/or activity of metalloproteases is reduced in the subject following administration of the inhibitor.

5. The method of claim 1, wherein the expression of inflammatory chemokines and cytokines selected in the group comprising CCL2, CXCL1, IL-1B, IL-8 is reduced in the subject following administration of the inhibitor.

6. The method of claim 1, wherein the expression of chromatin modulators DMT3A and TET2 is increased in the subject following administration of the inhibitor.

7. The method according to one of the claims from 1 to 6, wherein said inhibitor is a nucleic acid having a sequence comprising tggcagct (SEQ ID NO: 2), wherein the nucleic acid comprises at least one locked nucleic acid (LNA) modification.

8. The method according to one of the claims from 1 to 7, wherein the nucleic acid comprises LNA modifications at positions 7 and 8 of tggcagct (SEQ ID NO: 2).

9. The method according to one of the claims from 1 to 8, wherein the nucleic acid is CTTcaACtgGCAgCT (SEQ ID NO: 8), where capital letters are LNA modified and lower case are unmodified.
